# EUROPÄISCHE PATENTSCHRIFT

(11) **EP 0 557 576 B1**
(45) Veröffentlichungstag und Bekanntmachung des Hinweises auf die Patenterteilung: **17.12.1997**
(21) Anmeldenummer: 92115742.6
(22) Anmeldetag: 15.09.1992
(51) Int. Cl.: A61B 17/22, A61M 25/00, A61M 25/01

(54) **Vorrichtung zum Vereinzeln von Fremdkörpern in einem länglichen Organ eines Lebewesens**
Device to isolate foreign bodies in tubular body organs
Dispositif pour isoler des corps étrangers dans un organe tubulaire du corps humain

(30) Priorität: 28.02.1992 IT MI920449
(43) Veröffentlichungstag der Anmeldung: 01.09.1993
(73) Patentinhaber: Dormia, Guido, 22053 Lecco (IT)
(72) Erfinder: Dormia, Guido, 22053 Lecco (IT)
(74) Vertreter: Incollingo, Italo

(56) Entgegenhaltungen:
- EP-A- 0 180 348
- EP-A- 0 287 763
- EP-A- 0 317 091
- WO-A-90/03760
- WO-A-93/02721
- DE-U- 8 703 579
- US-A- 4 111 190

## Beschreibung

Die Erfindung betrifft eine Fremdkörpervereinzelungs-Vorrichtung für ein längliches Organ eines Lebewesens, wie den Ureter.

Zum Zerstören von Steinen in Organen eines Lebewesens, wie insbesondere den Nieren eines menschlichen Körpers, werden geeignete Vorrichtungen zum Aussenden von Druckwellen verwendet, nämlich sogenannte Lithotripter. Die Druckwellen sorgen dafür, daß die Steine zu Fragmenten zerbrochen werden. Die Steine werden dann durch den Ureter, die Blase und schließlich die Urethra aus dem Körper ausgeschieden.

Es hat sich herausgestellt, daß in vielen Fällen das Entfernen der Steine schwierig und schmerzhaft sowie ohne Operation unmöglich ist. Dies liegt daran, daß die Fragmente dazu tendieren, nahezu gleichzeitig sich durch den Harnleiter zu bewegen und in diesem einen Verschluß zu bilden. Es wurde beobachtet, daß der Verschluß sich häufig am Ende des Harnleiters bildet, Wo dieser in die Blase übergeht, da der Harnleiter in diesem Endabschnitt verengt ist.

Hieraus ergibt sich wiederum, daß der innerhalb der Niere gebildete Urin aufgrund des Verschlusses nicht aus dem Harnleiter ausgeschieden werden kann, da sich der Verschluß wie ein "Propfen" verhält und sich daher die Flüssigkeit im Harnleiter ansammelt, die Harnleiterwände anschwellen und beim Patienten starke Schmerzgefühle verursacht werden. Darüber hinaus entsteht ein Rückstau, der zu einer Schädigung der Niere führen kann.

Dies macht eine Operation des Patienten notwendig, um derartige Verschlüsse zu entfernen. Eine solche Operation kann endoskopisch vorgenommen werden. Hierbei wird zunächst ein Endoskop mit einer optischen Faser eingeführt, um den Verschluß zu lokalisieren. Dann wird der Verschluß mittels eines anderen Schlauches oder Katheters entfernt. Es kann passieren, daß ein solcher Verschluß sehr kompakt ist, so daß das Harnleitergewebe aufgeschnitten werden muß, um den Durchgang zu weiten und die Fragmente entfernen zu können.

Es wurde beobachtet, daß von den Fragmenten, die durch Zertrümmern der Steine entstanden sind, solche am ehesten einen Verschluß innerhalb des Harnleiters verursachen, die Staubkorngröße haben. Derartige Fragmente sammeln sich leichter an und binden sich leichter aneinander als die größeren und gröberen Fragmente.

Die Entfernung von Nierensteinen kann daher langandauernd und mühsam sein, was zu Beschwerden des Patienten führt.

Aus der EP 287 763 A2 ist eine Vorrichtung zum Offenhalten eines Ureterotomieschnitts bekannt. Bei der Ureterotomie handelt es sich um eine operative Längsspaltung des Harnleiters, insbesondere zur Behebung einer Stenose des Harnleiters. Es ist notwendig, die entsprechenden Schnitte im Harnleiter offenzuhalten. Hierzu sieht die EP 287 763 A2 eine entsprechende Vorrichtung mit einer im wesentlichen gestreckten Ureterotomieschiene vor, auf der ein Aufweitkörper verschiebbar aufsitzt, so daß er an den Ort der Stenose im Harnleiter angepaßt werden kann, wobei das distale und das proximale Ende der Ureterotomieschiene halbbogenförmig gebogen sind. Der Aufweitkörper weist einen Radius auf, der im wesentlichen dem des Harnleiters entspricht und beim Zwei- bis Dreifachen der Stärke der Schiene selbst liegt. Die Schiene weist im in der Niere liegenden Bereich eine Verdickung auf, die verhindert, daß beim Herausziehen der Schiene der Aufweitkörper von der Schiene abrutscht. Schiene und Aufweitkörper bestehen in der Regel aus geeignetem Kunststoffmaterial. Die Vereinzelung von Steinfragmenten wird in der EP-A-287 763 nicht erwähnt.

Der Erfindung liegt daher die Aufgabe zugrunde, eine Vorrichtung zu schaffen, die die Bildung von Verschlüssen im Harnleiter vermeidet und mittels der sich gegebenenfalls, falls sich dennoch im Einzelfall ein Verschluß bilden sollte, dieser ohne Schwierigkeiten und ohne Schmerzen für den Patienten entfernen läßt.

Erfindungsgemäß wird die genannte Aufgabe bei einer Vorrichtung der eingangs genannten Art durch die Merkmale des Anspruchs 1 gelöst.

Über die Steinvereinzelung hinaus weist die erfindungsgemäße Vorrichtung den Vorteil auf, daß sie gleichzeitig eine einen Urinrückfluß verhindernde Ureterschiene bildet, wodurch Reinfektionen vermieden werden. Die Länge der erfindungsgemäßen Vorrichtung kann in geeigneter Weise an unterschiedliche Ureterlängen angepaßt sein. Sowohl das längliche Element als auch die Schikanen können unterschiedliche Durchmesser aufweisen. In bevorzugter Weise kann die Vorrichtung auch als Endoprothese mit bis aus der Harnblase ragendem Ende ausgebildet sein.

Die Schikanen können in Form von Abstand zueinander aufweisenden, am länglichen drahtförmigen Element vorgesehenen Verdickungen ausgebildet sein. Die Verdickungen können kugelförmig, halbkugelförmig sein. Sie können die Form von zwei an ihrer Basis verbundenen Kegeln aufweisen. Sie können auch scheiben- oder tropfenförmig ausgeformt sein.

Durch die Ausbildung der erfindungsgemäßen Vorrichtung wird zwischen zwei Schikanen oder Verdickungen ein Freiraum für die zeitweilige Aufnahme von Steinfragmenten belassen. Hierdurch wird eine zuverlässige Vereinzelung der Steinfragmente über die Länge der erfindungsgemäßen Vorrichtung und damit die Länge des länglichen Organs, wie insbesondere des Ureters hin erreicht. Durch diese Vereinzelung wird die Bildung von Verschlüssen nahezu ausgeschlossen.

Die Erfindung sieht vor, daß das längliche Element aus Metalldraht besteht. Eine vorteilhafte, patientenschonende Weiterbildung insbesondere sieht vor, daß das längliche Element aus einer Formgedächtnislegierung (Memory-Metall) besteht.

In Weiterbildung kann vorgesehen sein, daß die Stirnseiten des länglichen Elements mit Verdickungen versehen sind, insbesondere wenn das vordere und/oder rückwärtige Ende des länglichen Elements spiralförmig gebogen ist. Hierdurch werden Verletzungen des Nierensystems bzw. der Harnblasenwand zuverlässig ausgeschlossen.

Eine Weiterbildung sieht vor, daß die Schikanen und/oder Verdickungen röntgendichtes Material enthalten. Ein derartiges Material ist beispielsweise Wismuthkarbonat.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnung im einzelnen erläutert ist. Dabei zeigt:
- Figur 1: eine schematische Darstellung der Harnorgane eines menschlichen Körpers mit einem in der Niere dargestellten Stein;
- Figur 2: einen durch Fragmente des in der Figur 1 gezeigten Steins gebildeten Verschluß am unteren Ende des Ureters;
- Figur 3: eine bevorzugte Ausgestaltung der erfindungsgemäßen Vorrichtung in den Harnorganen des menschlichen Körpers mit einem Einführkatheter;
- Figur 4: die erfindungsgemäße Vorrichtung in Arbeitsposition vor dem Zerstören des Steines;
- Figur 5: die erfindungsgemäße Vorrichtung während ihrer Funktion nach Aufbrechen des Steines;
- Figur 6: eine schematische Darstellung, wie eine im Ureter gebildete Anhäufung von Steinfragmenten entfernt werden kann;
- Figur 7: eine vergrößerte Darstellung des oberen Bereichs der Figur 4;
- Figur 8: eine vergrößerte Darstellung des Mittelbereichs der Figur 5;
- Figur 9: eine naturnahe Darstellung der Harnorgane mit einem Verschluß im linken Ureter, wie er durch die im rechten Ureter liegende erfindungsgemäße Vorrichtung durch Vereinzelung der Steinfragmente verhindert wird.

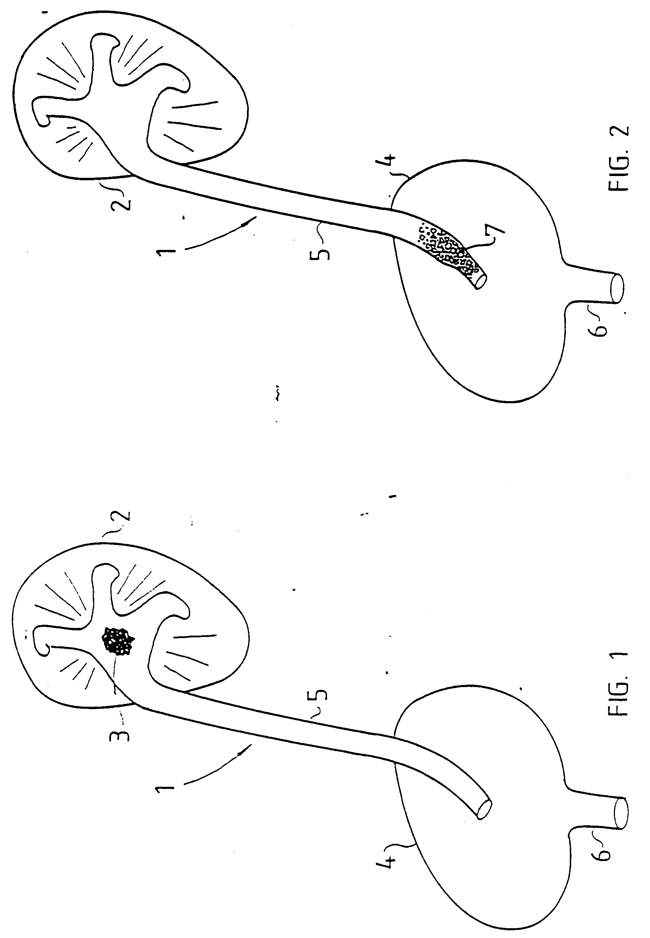

Figur 1 zeigt die Harnorgane 1 eines menschlichen Körpers. Ein erstes Organ ist die Niere 2. In der Niere ist ein Nierenstein 3 dargestellt. Die Niere 2 ist mit der Harnblase (Vesica Urinalis) über den Ureter 5 verbunden, der etwas in die Harnblase 4 hineinragt. Aus der Harnblase 4 führt die Urethra 6.

Nach Zerstören eines Steins in einem menschlichen Organ, wie eines Nierensteins in der Niere kann es vorkommen, daß die aus dem Organ durch einen entsprechenden Leiter, wie hier dem Ureter abgehenden Steinfragmente 7 sich im Leiter sammeln und diesen verstopfen, also einen Verschluß bilden. Insbesondere können feine Staubkornteilchen große Steinfragmente wieder fest miteinander verkleben. Ein solcher Verschluß ist nur mühsam zu entfernen, gegebenenfalls nur operativ.

Hier setzt die Erfindung an.

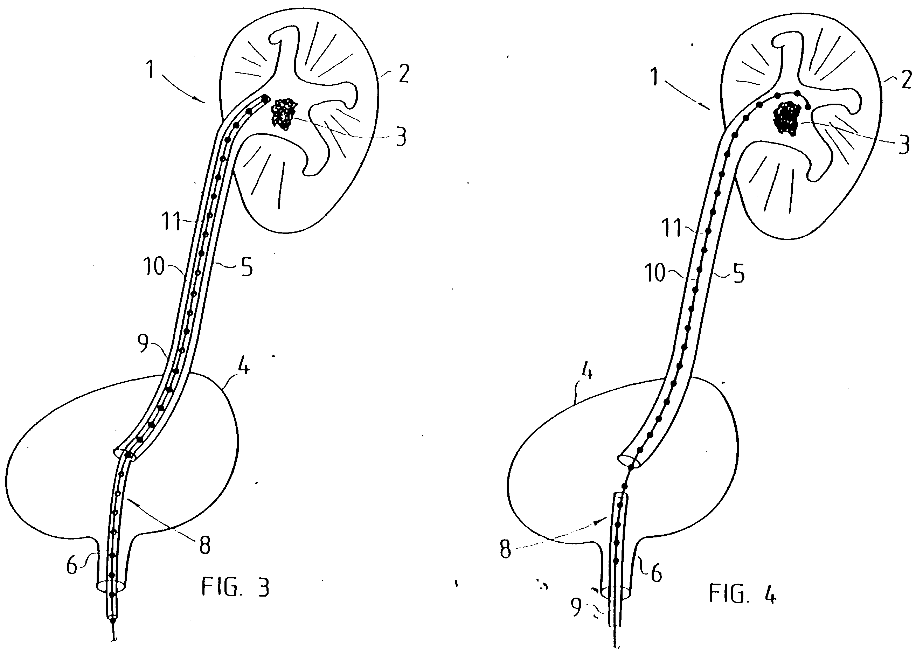

Die erfindungsgemäße Vorrichtung weist im dargestellten Ausführungsbeispiel einen Draht 10 mit auf diesem mit Abstand zueinander angeordneten Schikanen in Form von kugelförmigen Verdickungen 11 auf. Der Draht besteht bevorzugt aus rostfreiem Stahl, welcher eine geeignete Steifigkeit und Biegsamkeit aufweist. Das längliche Element kann auch ein Massivteil sein. Statt dessen kann das Teil auch aus schraubenförmig verbundenen Einzellitzen bestehen. Das so gebildete längliche Teil kann gegebenenfalls auch aufgrund seiner Eigensteifigkeit statt der in der Figur 3 dargestellten gestreckten Form über einen Teil seiner Länge - nämlich im Ureter - in Schraubenwindungen geführt sein, die einen endlichen Abstand zueinander aufweisen, um so den Ureter, der einen muskulös-häutigen Schlauch bildet, weiter offen zu halten und im Inneren der genannten Schraubenwindungen einen freien Durchgang zu gewährleisten. Das längliche Element reicht von der Niere 2 bis außerhalb des menschlichen Körpers.

Die Schikanen 11 können wie in der dargestellten Ausführungsform kugelförmige Verdickungen auf dem gestreckten Verbindungsteil sein. Es können aber auch halbkugelförmige, scheibenförmige oder tropfenförmige Verdickungen sein. Die Verdickungen können auch die Form eines Doppelkegels, also die Form von zwei an ihrer Basis verbundenen Kegeln aufweisen. Die Verdickungen können ebenfalls aus den verschiedenartigsten Materialien bestehen, wie Metall, insbesondere rostfreiem Stahl, insbesondere wenn das längliche Verbindungselement ebenfalls aus rostfreiem Stahl besteht, Kunststoff oder dergleichen.

In bevorzugter Ausgestaltung bestehen die Schikanen aus auf dem länglichen Element in Abständen befestigten Kügelchen aus Acrylat oder Dimethylacrylat, dem jeweils Wismutkarbonat beigemischt ist, um die gewünschte Röntgendichtigkeit zu erreichen.

Insbesondere soweit das längliche Verbindungselement in der beschriebenen Weise schraubenförmig mit Abstand der einzelnen Schraubenwindungen geführt ist, kann hierfür vorzugsweise ein Metall aus einer Gedächtnislegierung verwendet werden, die so vorgespannt ist, daß das längliche Verbindungselement bei niedriger Einführtemperatur gestreckt ist und erst in seiner vorgespannten Hochtemperaturstellung die schraubenförmige Form annimmt.

Die erfindungsgemäße Vorrichtung aus länglichem Verbindungsteil mit auf diesem mit Abstand zueinander angeordneten Verdickungen wird vorzugsweise mittels eines Katheters 9 aus Kunststoff mit hoher Elastizität in den Ureter eingeführt, wie dies in der Figur 3 dargestellt ist. Der Katheter 9 selbst wird dabei, da er an seiner Vorderseite offen sein muß, damit er später wieder über die gelegte erfindungsgemäße Vorrichtung abgezogen werden kann, in Seldinger-Technik eingeführt.

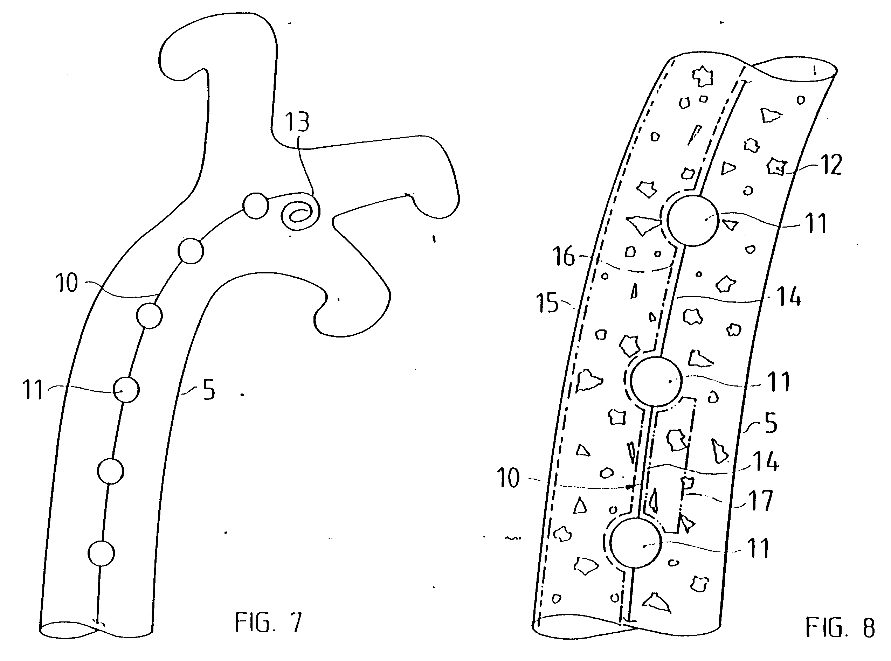

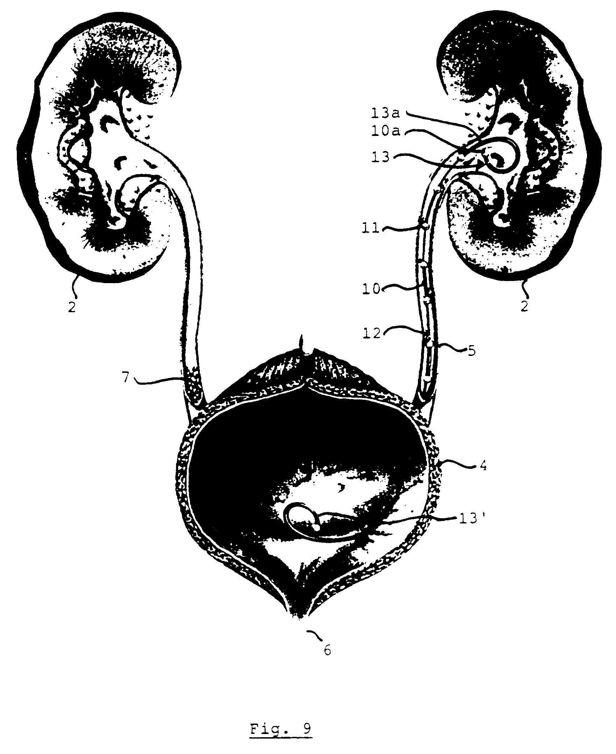

Nach Legen des Katheters kann durch diesen die erfindungsgemäße Vorrichtung mit dem länglichen Verbindungsteil 10 und den Verdickungen 11 eingeführt werden, bis deren vorderes Ende 13 in die Niere 2 gelangt. Wenn das vordere Ende 13 (Figur 7) dort aus dem Katheter 9 herausgeführt wird, so legt es sich zumindestens halbbogenförmig oder aber spiralig, um hier eine Rückhaltesicherung in der Niere insbesondere auch beim Herausziehen des Katheters zu bilden. Die Enden 13, 13' des länglichen Elements 10 können, soweit dieses aus Draht besteht, mit einer Kunststoffumhüllung 10a versehen sein. An den Stirnseiten können ebenfalls Verdickungen vorgesehen sein bzw. mindestens im Endbereich des länglichen Elements 10 ist ein Kunststoffüberzug 13a vorgesehen (Fig. 9).

Der Katheter 9 wird anschließend aus dem Ureter entfernt.

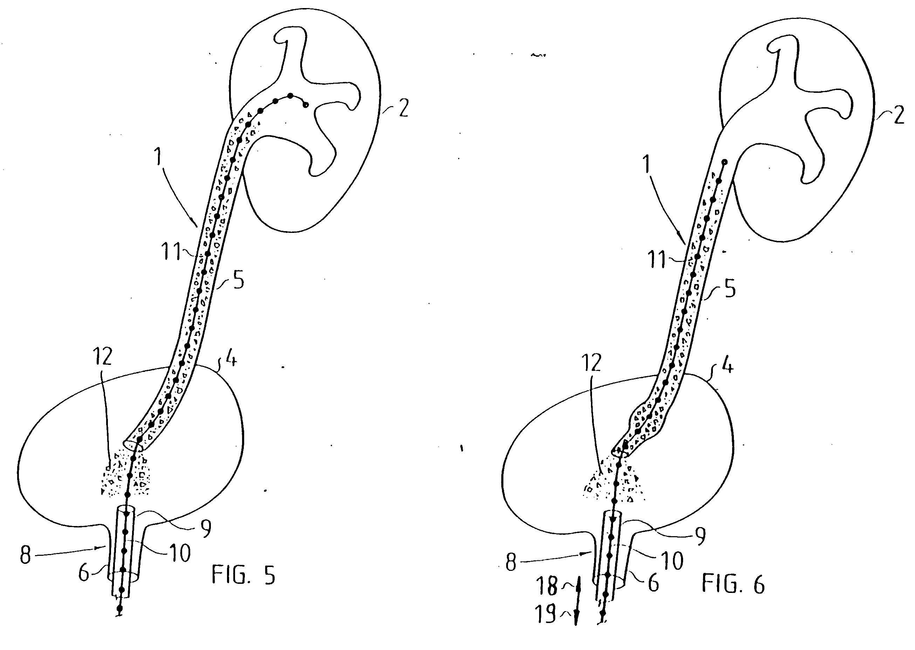

Sodann kann die Steinzertrümmerung mittels von einem Lithotripter erzeugter Druckwellen vorgenommen werden, woraufhin die einzelnen Fragmente 12 des Steins in der aus der Figur 5 ersichtlichen Weise durch in der Niere 2 gebildeten Urin aus dieser durch den Harnleiter 5 an der gelegten erfindungsgemäßen Vorrichtung vorbei zunächst in die Harnblase 4 ausgespült werden. Die einzelnen Steinfragmente rutschen dabei nicht in einem Paket durch den Ureter 5, sondern werden bei ihrer Bewegung durch diesen vereinzelt und zum Teil über die gesamte Länge der Vorrichtung hin in den Zwischenräumen zwischen den einzelnen Verdickungen 11, die regelmäßige Änderungen des freien Harnleiterquerschnitts bewirken, mehr oder minder kurz zurückgehalten, so daµ sie sich zunächst über die gesamte Länge der erfindungsgemäßen Vorrichtung verteilen.

Die Figur 8 zeigt, daß jedes Paar von aufeinanderfolgenden Verdickungen 11 und die Abschnitte von Draht 14, die sich dazwischen befinden, Bereiche bilden, durch die die Fragmente 12 vereinzelt werden, indem sie in diesen Bereichen zeitweilig gesammelt werden, so daß sie in ihren Bewegungen unterschiedlich verzögert werden. Einer dieser Bereiche wird durch eine geschlossene Strich- und Punktlinie symbolisiert und durch die Nummer 17 in Figur 8 bezeichnet.

Bei der in den Figuren dargestellten gestreckten Ausführungsform des länglichen Verbindungselementes 10 können nahe der Wandung des Ureters 5 etwa entlang der Linie 15 sich bewegende Fragmente 12 weitgehend ungehindert nach unten rutschen, wobei sie sich allerdings dort nicht linear bewegen, sondern teilweise auch weiter zum Inneren des Ureters 5 hin wandern. Dort, also nahe der erfindungsgemäßen Vorrichtung sich bewegende Steinfragmente, die etwa der Linie 16 folgen, werden durch die Schikanen 11 in ihrer Bewegung gegenüber den vorbeschriebenen Steinfragmenten verzögert und müssen im übrigen die Schikanen umgehen, so daß sie auch einen langen Weg haben. Auf diese Weise, abhängig von der besonderen Form des Drahtes, wird sich ein Teil der Fragmente schneller bewegen und einer kürzeren Route entlang des Harnleiters folgen, um die Blase 4 zuerst zu erreichen. Auf der anderen Seite wird sich ein anderer Teil der Fragmente langsamer bewegen und einer längeren Route folgen und daher das Innere der Blase 4 später erreichen.

Insgesamt wird hierdurch, wie sich überraschenderweise herausgestellt hat, eine sehr gute Vereinzelung der Steinfragmente über die gesamte Länge des Ureters und der erfindungsgemäßen Vorrichtung erreicht, wie dies insbesondere in der Figur 5 dargestellt ist. Die Bildung von Verschlüssen wird vermieden, da sich die Fragmente unterschiedlich und nicht gleichzeitig durch den Harnleiter bewegen. Dies ist in vivo sehr gut der Fig. 9 zu entnehmen.

Sollte dennoch in Einzelfällen am unteren Ende des Ureters eine Kumulation von Steinfragmenten erfolgen, wie dies in der Figur 6 dargestellt ist, so kann diese leicht aufgelöst werden, indem die erfindungsgemäße Vorrichtung von außen her leicht axial hin- und herbewegt wird, wodurch die angesammelten Steinfragmente aus der unteren Uretermündung in die Harnblase bewegt werden können. Diese Aufund Abbewegungen des Drahtes verursachen eine Auflockerung der den Verschluß bildenden Fragmente und befreien folglich den Durchgang durch den Harnleiter 5. Diese Vorgangsweise ist aufgrund der Tatsache möglich, wie bereits festgestellt, daß der Draht aus einem Material hergestellt ist, welches eine gewisse Biegsamkeit besitzt, was eine leichte Einführung zusammen mit dem Katheter in den menschlichen Körper erlaubt, aber zur selben Zeit eine gewisse Steifheit vorhanden ist, die es ermöglicht, ihn innerhalb des Harnleiters zu schieben, ohne daß er wellig gestaucht wird.

Es hat sich herausgestellt, daß durch die erfindungsgemäße Vorrichtung die Gefahr von Clusterbildungen im Ureter, insbesondere im unteren Abschnitt des Ureters, wesentlich reduziert werden und Ansammlungen von Steinfragmenten dort nur noch in wenigen Fällen im unteren Prozentbereich auftreten. Es hat sich weiter herausgestellt, daß diese Ansammlungen in der erfindungsgemäßen Form entfernt werden können, so daß größere Eingriffe, wie sie bisher bei den oft auftretenden Clusterbildungen notwendig waren und oft in Form einer Operation durchgeführt werden mußten, vollständig vermieden werden können.

Die Anwendung der erfindungsgemäßen Vorrichtung ist nicht nur auf Steine, die sich innerhalb der Niere bilden, begrenzt, sondern auch auf Steine, die sich innerhalb der Blase bilden, möglich. Anstatt die Vorrichtung in den Harnleiter 5 einzuführen, ist es in diesem Falle ausreichend, diese in die Harnröhre 6 einzuführen, bevor man die Steine durch Anwendung von Druckwellen zerbricht. Anschließend wird die Vorrichtung, wie vorausgehend beschrieben, zur Entfernung der Steinfragmente aus der Niere benutzt.

## Patentansprüche

1. Fremdkörpervereinzelung-Vorrichtung für ein längliches Organ eines Lebewesens, wie den Ureter, mit einem länglichen, drahtförmigen Element (10) aus Metalldraht, das mit Schikanen (11) versehen ist und dessen vorderes, distales und rückwärtiges, proximales Ende (13, 13') zumindest halbbogenförmig gebogen sind.

2. Vorrichtung nach Anspruch 1, gekennzeichnet durch einen das längliche, mit Schikanen versehene Element umgebenden Katheter (9).

3. Vorrichtung nach Anspruch 1 oder 2, dadurch gekennzeichnet, daß die Schikanen (11) durch Verdickungen auf dem länglichen Element gebildet sind.

4. Vorrichtung nach einem der Ansprüche 1 bis 3, dadurch gekennzeichnet, daß das längliche Element (10) aus einer Formgedächtnislegierung besteht.

5. Vorrichtung nach einem der Ansprüche 1 bis 4, dadurch gekennzeichnet, daß mindestens im Endbereich des länglichen Elements (10) ein Kunststoffüberzug (13a) vorgesehen ist.

6. Vorrichtung nach einem der vorangehenden Ansprüche, dadurch gekennzeichnet, daß das längliche Element (10) ein gestreckter Massivkörper ist.

7. Vorrichtung nach einem der Ansprüche 1 bis 5, dadurch gekennzeichnet, daß das längliche Element (10) aus schraubenförmig gewickelter Litze besteht, wobei die Windungen aneinander anliegen.

8. Vorrichtung nach einem der vorangehenden Ansprüche, dadurch gekennzeichnet, daß das längliche Element (10) in Abstand zueinander aufweisenden Windungen geführt ist.

9. Vorrichtung nach einem der vorangehenden Ansprüche, dadurch gekennzeichnet, daß das vordere und/oder rückwärtige Ende (13, 13') des länglichen Elements (10) spiralförmig gebogen ist.

10. Vorrichtung nach einem der vorangehenden Ansprüche, dadurch gekennzeichnet, daß die Stirnseiten des länglichen Elements (10) mit Verdickungen versehen sind.

11. Vorrichtung nach einem der vorangehenden Ansprüche, dadurch gekennzeichnet, daß die Schikanen (11) röntgendichtes Material enthalten.

## Claims

1. Apparatus for separating foreign bodies in an elongated organ of a living organism such as the ureter, with an elongated element (10) in the form of a wire of metal, which is provided with daffles (11) and the front distal and the rear proximal ends (13, 13') of which are at least bent in a semiarcuate form.

2. Apparatus according to claim 1, characterized by a catheter (9) surrounding the elongated element provided with baffles.

3. Apparatus according to claim 1 or 2, characterized in that the baffles (11) are formed by thickened portions on the elongated element.

4. Apparatus according to one of the claims 1 to 3, characterized in that the elongated element (10) consists of a form-memory alloy.

5. Apparatus according to one of the claims 1 to 4, characterized in that at least in the terminal zone the elongatated element (10) is provided with a coating (13a) of synthetic material.

6. Apparatus according to one of the preceding claims, characterized in that the elongated element (10) is a stretched massive body.

7. Apparatus according to one of the claims 1 to 5, characterized in that the element (10) consists of a strand wound in screw form wherein the windings are disposed one near the others.

8. Apparatus according to one of the preceding claims, characterized in that the windings of the elongated element (10) are distant from each other.

9. Apparatus according to one of the preceding claims, characterized in that the proximal and/or distal ends (13, 13') of the elongated element (10) are bent in form of a spiral.

10. Apparatus according to one of the preceding claims, characterized in that the front sides of the elongated element (10) are provided with thickenings.

11. Apparatus according to one of the preceding claims, characterized in that the baffles (11) contain röntgen opaque material.

## Revendications

1. Dispositif pour eliminer des corps étrangers d'un organ allongé d'un organism vivant telle que l'urètre, ayant un élément (10) allongé à forme de fil de métal, lequel est pourvu de protubérances et présente les extremités (13, 13') anterieure voisine et posterieure lointaine pliées au moins en forme demi-arquée.

2. Dispositif selon la revendication 1, characterisé par un cathéter (9) environnant l'élément allongé pourvu de protubérances.

3. Dispositif selon la revendication 1 ou 2, characterisé en ce que les protubérances (11) sont formées par des grossissements sur l'élément allongé.

4. Dispositif selon une des revendications de 1 à 3, characterisé en ce que l'élément allongé (10) consiste d'un alliage avec memorie de forme.

5. Dispositif selon une des revendications de 1 à 4, characterisé en ce que un revêtement en matiére synthetique (13a) est pourvu au moins dans la zone terminale de l'élément allongé (10).

6. Dispositif selon une des revendications précédentes, characterisé en ce que l'élément allongé (10) est un corps massif étiré.

7. Dispositif selon une des revendications de 1 à 5, characterisé en ce que l'élément allongé (10) consiste d'un fil enroulé à forme de vis, les spires étant disposées les unes à coté des autres.

8. Dispositif selon une des revendications précédentes, characterisé en ce que l'élément allongé (10) a des spires lesquelles présentent une distance entre elles.

9. Dispositif selon une des revendications précédentes, characterisé en ce que l'extremité anterieure et/ou posterieure (13, 13') de l'élémént allongé (10) est pliée à forme de spirale.

10. Dispositif selon une des revendications précédentes, characterisé en ce que les cotés frontaux de l'élément allongé (10) sont pourvus de grossissements.

11. Dispositif selon une des revendications précédentes, characterisé en ce que les protubérances (11) contiennent du material röntgen opaque.
